# EUROPEAN PATENT APPLICATION

(11) **EP 2 556 850 A1**
(43) Date of publication of application: **13.02.2013**
(21) Application number: 12179051.3
(22) Date of filing: 02.08.2012
(51) Int. Cl.: A61M 1/36, A61M 5/32

(54) **Detection and guide systems for accessing blood vessels**

(30) Priority: 08.08.2011 US 201113205474
(71) Applicant: Teitelbaum, George P., Santa Monica, CA 90402 (US); Shaolian, Samuel M., Newport Beach, CA 92660 (US); Pool, Scott L., Laguna Hills, California 92653 (US)
(72) Inventor: Teitelbaum, George P., Santa Monica, CA 90402 (US); Shaolian, Samuel M., Newport Beach, CA 92660 (US); Pool, Scott L., Laguna Hills, California 92653 (US)
(74) Representative: Bradley, Adrian

(57) **Abstract**

A detection and guide system provides access to desired blood vessel locations. An implant formed of a biocompatible material is implanted within a mammalian body proximate an outer surface of a blood vessel. The detection and guide system may be configured to be positioned outside the mammalian body to detect the implant and to guide a needle to the desired entry site of the biological boundary structure. A needle guide may be coupled to a housing of the detection and guide system and directed at an angle such that positioning the implant detector over the implant aligns an opening through the needle guide with the desired entry site. The detection and guide system may, for example, provide metal detection of a metallic implant or magnetic detection of a magnetic implant.

## Description

### Technical Field

This disclosure relates to vascular access and relates to systems and methods for locating a desired entry site into a biological boundary structure.

### Background Information

Repetitive vascular access is used for treatments such as prolonged intravenous chemotherapy protocols and venous hemodialysis.

Among the patients needing repeated vascular access are chemotherapy patients. A large number of chemotherapeutic agents are infused intravenously over multiple cycles during the treatment of a wide variety of neoplasms. Because many of these agents can cause pain and vessel thrombosis and sclerosis, these chemotherapeutic agents are generally infused into a larger central vein by means of a peripherally inserted central catheter or "PIC line" (e.g., a size 4 French (F) or lower diameter catheter may be inserted, usually, into a basilic or cephalic vein of the upper extremity), the distal tip of which is advanced into a central vein such as the superior vena cava. However, the PIC line can occlude and can cause phlebitis with propagation of clot centrally that may require long-term anticoagulation therapy as well as removal of the PIC line. In addition, because the infusion port of a PIC lines is outside the skin, it is possible for infection to track along the course of the catheter.

Chemotherapeutic infusions can also be accomplished through infusion port catheters where injectable infusion ports are implanted subcutaneously, usually in the upper chest region. The distal catheters of infusion port catheter devices are usually inserted into the superior vena cava via a puncture site within the subclavian or jugular veins. Infusion port catheters are also subject to occlusion and phlebitis. Also, stenoses (narrowing) can develop at the catheter insertion site within the subclavian or jugular vein.

Dialysis patients may also need repeated vascular access. Currently, more than 350,000 Americans are undergoing hemodialysis approximately three times a week for chronic renal failure. Although this is often accomplished using a surgically created upper extremity arteriovenous (AV) fistula (a polytetrafluoroethylene (PTFE) graft connecting an artery and a vein in the forearm or upper arm which has replaced the Scribner shunt), at times peritoneal dialysis or venous hemodialysis are used. Problems associated with AV fistula hemodialysis include frequent shunt thrombosis that requires a semi-emergent thrombolysis/thrombectomy +/- balloon angioplasty procedure performed by a vascular interventionist. This type of costly intervention may be required two to four times per year. These AV fistulas may also be associated with anastomotic stenoses as well as more central venous stenoses. Dialysis shunts may ultimately fail after several years of use, thus progressively limiting future options for creating a new hemodialysis access site. Peritoneal dialysis is generally less convenient than hemodialysis and entails the risk of serious or life-threatening peritonitis.

Venous hemodialysis has an advantage of minimally invasive access catheter insertion (with no open surgical procedure). However, currently its disadvantages include thrombophlebitis, thromboembolization, and entry site venous stenoses (which are significantly more difficult to treat than arterial stenoses). Once such a stenosis develops in the subclavian vein, attempts at using the ipsilateral upper extremity for the surgical creation of an AV fistula for hemodialysis are frequently unsuccessful. Typically, venous hemodialysis requires an indwelling approximately 14 F (French catheter scale, in which the diameter in millimeters can be determined by dividing the French size by three) dual lumen (one lumen for withdrawing blood and the other for reinjection of the blood returning from the hemodialysis unit) hemodialysis catheter that has its proximal ports protruding from the skin surface. This long-term surface access increases the risk of infection tracking from the skin surface along the catheter shaft and into the deep perivenous tissues and even into the intravascular space (an AV hemodialysis fistula is entirely subcutaneous).

### Summary

In one embodiment, a system for accessing a biological boundary structure at a desired entry site within a mammalian body includes an implant and a detection and guide system. The implant may be formed of a biocompatible material and configured to be implanted within the mammalian body proximate an outer surface of the biological boundary structure. The detection and guide system may be configured to be positioned outside the mammalian body to detect the implant and guide a needle to the desired entry site of the biological boundary structure. The detection system may include an implant detector and a needle guide. The implant detector may be positioned within a housing of the detection and guide system and may be configured to detect a detectable material of the implant. The needle guide may be coupled to the housing of the detection and guide system and directed at an angle such that positioning the implant detector over the implant aligns an opening through the needle guide with the desired entry site.

In one embodiment, a system for accessing a biological boundary structure at a desired entry site within a mammalian body includes one or more metal detector coils within a coil housing. The one or more metal detector coils are configured to detect a metallic implant within the mammalian body. The system also includes a guide canula attached to the coil housing at an angle such that placement of the one or more metal detector coils over the metallic implant aligns an opening through the guide canula with the desired entry site.

In one embodiment, a system for accessing a biological boundary structure at a desired entry site within a mammalian body includes an implant and a detection and guide system. The implant may include a magnetic material that creates a detectable magnetic field. The detection and guide system may include a compass and a needle guide. The compass may be configured to detect the magnetic material of the implant. The compass may include a locator configured to be able to shift position relative to the housing and needle guide of the detection and guide system. The locator may shift to align with a magnetic field of the magnetic material of the implant. The needle guide may be coupled to the housing of the detection and guide system and directed at an angle such that positioning the implant detector over the implant aligns an opening through the needle guide with the desired entry site.

Additional aspects and advantages will be apparent from the following detailed description of preferred embodiments, which proceeds with reference to the accompanying drawings.

### Brief Description of the Drawings

FIG. 1 is a perspective view of a funnel-shaped subcutaneous conduit shown as affixed to a blood vessel according to one embodiment.

FIG. 2 is a cross-section view of the funnel-shaped subcutaneous conduit shown in FIG. 1 implanted in a patient according to one embodiment.

FIG. 3A is a cross-section view of a sheathed needle accessing a blood vessel through the funnel-shaped subcutaneous conduit according to one embodiment.

FIG. 3B is a cross-section of the funnel-shaped subcutaneous conduit in a coapted state according to one embodiment.

FIGS. 4A, 4B, 4C, 4D, 4E, and 4F are cross-section views illustrating a method for inserting the funnel-shaped subcutaneous conduit into a patient according to one embodiment.

FIGS. 5A and 5B are enlarged cross-section views of a hypotube positioned adjacent to a tab of the funnel-shaped subcutaneous conduit according to one embodiment.

FIG. 6 is a perspective view of a sluice-shaped or "dust pan" subcutaneous conduit according to one embodiment.

FIGS. 7A, 7B, 7C, 7D, and 7E schematically illustrate an elongated funnel-shaped subcutaneous conduit according to certain embodiments.

FIGS. 8A, 8B, and 8C schematically illustrate a detection and guide system for accessing a blood vessel at a desired entry site according to one embodiment.

FIG. 9 is a perspective view of a detection and guide system for accessing a blood vessel at a desired entry site according to another embodiment.

FIGS. 10A, 10B, and 10C are side, top, and perspective views, respectively, of a detection and guide system for accessing a blood vessel at a desired entry site according to one embodiment.

FIGS. 11A and 11B are perspective views of a detectable implant of a system for accessing a blood vessel at a desired entry site according to one embodiment.

FIG. 12A illustrates a system for implanting a detectable implant according to one embodiment

FIG. 12B is a side sectional view of a detectable implant after successful insertion according to one embodiment.

FIG. 13 is side view of system for accessing a blood vessel at a desired entry site according to another embodiment.

### Detailed Description

Intravenous chemotherapy infusion and venous hemodialysis may be significantly improved if one could avoid the use of in-dwelling catheters to accomplish these techniques. If one could rapidly and safely access a larger central vein, while minimizing trauma to this vessel, and could reliably access such a vessel repeatedly over the course of months to years, one could avoid or limit the problems of venous entry site stenosis, phlebitis, infusion catheter occlusion, and central propagation of clot. If such repeated but temporary central venous catheterization could be conducted by non-physician personnel, such as at a chemotherapy/oncology or hemodialysis outpatient clinic, with a high probability of successful venous access and low risk of complications, such an improvement would make such venous therapy clinically successful.

Embodiments described herein include a subcutaneous needle conduit that attaches to the external adventitial layer of larger veins, arteries, or other biological boundary structures (as discussed below). The subcutaneous conduit can be easily located beneath the skin surface using, for example, tactile sensation, magnetism, metal detection, detection of a signal emitted from a minute transponder, detection of light emission (such as from fluorescent excitation or induced by heat), or through other detection methods.

After a user (e.g., a nurse, technician, or other medical practitioner) locates the subcutaneous conduit, the user may prepare and drape the skin area over the subcutaneous conduit's entry location in a sterile fashion. The user can then advance a sheathed metal needle through the skin and into the subcutaneous conduit. In certain embodiments, the inner lining of the subcutaneous conduit is adapted to prevent or limit perforation by the sharp needle tip, such as by incorporation of perforation resistant material such as Kevlar^{®}, another ballistic plastic, metal, or an appropriate composite material that provides armoring. The user may apply suction to the needle as it is advanced until back flow of blood through the needle is realized. At this point, the user advances the plastic sheath (e.g., composed of PVA, nylon, polyethylene, PVC, polyurethane, or the like, with or without braiding) off of the needle and into the more central venous circulation where it acts, for example, as a chemotherapy infusion catheter or a catheter for the withdrawal or reinjection of blood for hemodialysis. Once the drug infusion or hemodialysis session is completed, the user may remove the catheter. Local manual pressure may be applied to the entry site to ensure minimal bleeding from the zone of venous puncture.

Reference is now made to the figures in which like reference numerals refer to like elements. For clarity, the first digit of a reference numeral indicates the figure number in which the corresponding element is first used. In the following description, numerous specific details are set forth in order to provide a thorough understanding of the present disclosure. However, persons skilled in the art will recognize that certain embodiments can be practiced without one or more of the specific details or with certain alternative equivalent components, materials, and/or methods to those described herein. In other instances, well-known components and methods have not been described in detail so as not to unnecessarily obscure aspects of the present disclosure. Furthermore, the described features, structures, or characteristics may be combined in any suitable manner in one or more embodiments.

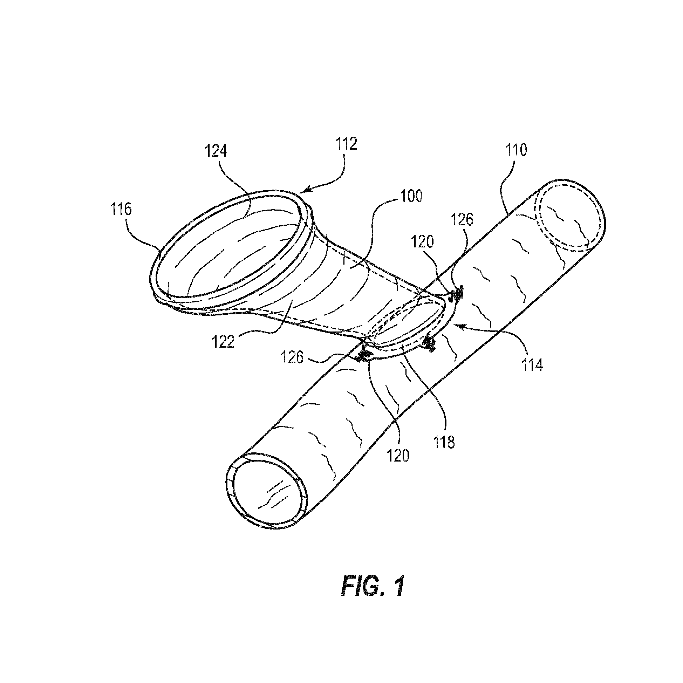

FIG. 1 is a perspective view of a funnel-shaped subcutaneous conduit 100 shown as affixed to a blood vessel 110 according to one embodiment. The funnel-shaped subcutaneous conduit 100 includes a proximal end 112 and a distal end 114. The proximal end 112 includes a proximal ring 116 reinforced with a rigid material such as titanium, Nitinol^{®}, stainless steel, or other material that maintains a desired shape and size of an entry location into the funnel-shaped subcutaneous conduit 100. The circumference of the proximal ring 116 may be selected to provide quick detection below a patient's skin and to provide a sufficient target area for passing a sheathed needle through the patient's skin for access to the blood vessel 110 through the funnel-shaped subcutaneous conduit 100. In one embodiment, a diameter of the proximal ring 116 is in a range between about 1 centimeter (cm) and about 3 cm. However, larger or smaller diameters may also be used. For example, in another embodiment, the diameter of the proximal ring 116 is about 4 cm. The proximal ring 116 also provides increased stiffness to aid in the location of the proximal end 112 of the funnel-shaped subcutaneous conduit 100 by a user using tactile sensation.

The funnel-shaped subcutaneous conduit 100 tapers down from the proximal end 112 to the distal end 114 to guide a needle to a target location at the blood vessel 110. In certain embodiments, an opening 118 in the distal end 114 has a diameter selected to be about the diameter of the particular blood vessel 110 targeted for access. For example, in one embodiment, a diameter of the distal end 114 of the funnel-shaped subcutaneous conduit 100 is in a range between about 8 millimeters (mm) and about 20 mm. The diameter of the opening 118 in the distal end 114 may be larger or smaller than this range. For example, depending on the size of the targeted blood vessel, the diameter of the diameter 118 of the distal end 114 may be as large as 30 mm or 40 mm. Further, in certain embodiments, the opening 118 in the distal end 114 may be larger than the diameter of the particular blood vessel 110 target for access.

As shown in FIG. 1, the distal end 114 may have two, three, four or more tabs 120 (three shown) of material spaced around the opening 118 of the distal end 114. The tabs 120 can serve as anchoring points for attaching the funnel-shaped subcutaneous conduit 100 to the blood vessel 110 or other biological boundary structure. In FIG. 1, the tabs 120 are shown with sutures or helical wires 124 (discussed below) used to affix the funnel-shaped subcutaneous conduit 100 to the adventitial layer of the blood vessel 110.

An external surface 122 of the funnel-shaped subcutaneous conduit 100 may, for example, include an adhesion resistant plastic (such as PTFE), a hydrophilic surface layer, an animal-derived material such as pericardium, or the like. The funnel-shaped subcutaneous conduit 100 may also include an inner lining 124 of, for example, a ballistic plastic, metal or similar material to prevent or limit perforation by an entry needle. Additional shape and/or body may be imparted to the funnel-shaped subcutaneous conduit 100 and the proximal ring 116, according to certain embodiments, by incorporating a layer of hydrogel within the needle-conducting portion (e.g., within the inner lining 124 of the funnel-shaped subcutaneous conduit 100) that swells with the absorption of adjacent water once the funnel-shaped subcutaneous conduit 100 has been deployed or implanted within a patient.

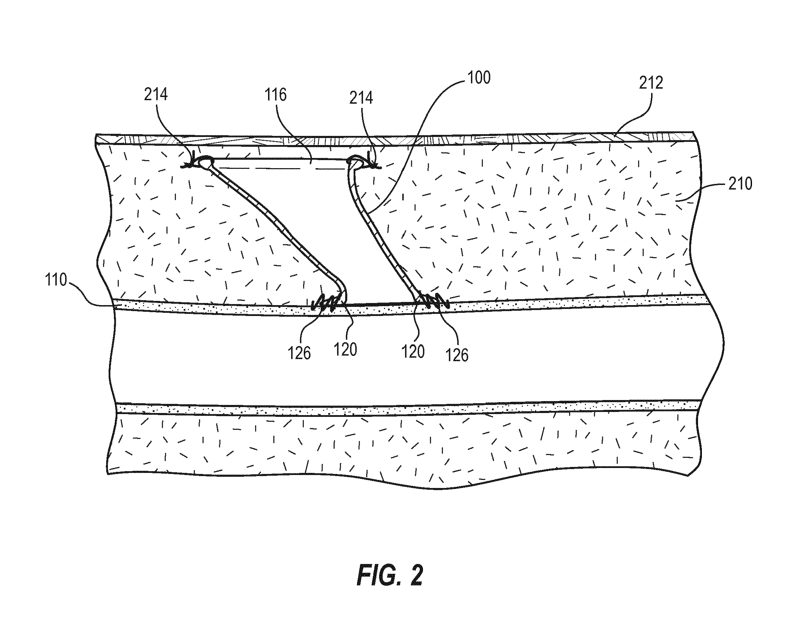

FIG. 2 is a cross-section view of the funnel-shaped subcutaneous conduit 100 shown in FIG. 1 implanted in a patient according to one embodiment. The funnel-shaped subcutaneous conduit 100 may be sized and configured for implantation within subcutaneous tissues 210 so as to provide a needle passageway from a location near the patient's skin 212 to the blood vessel 110. As shown in FIG. 2, helical wires 126 may be used to attach the tabs 120 to the external adventitial layer of the blood vessel 110. As further shown in FIG. 2, certain embodiments may also include one or more sutures 214 placed near the proximal ring 116 or at other locations on the funnel-shaped subcutaneous conduit 100. For example, the funnel-shaped subcutaneous conduit 100 may include a plurality of minute eyelets (not shown) around the periphery of the proximal ring 116 that allow the passage of the sutures 214 to secure the funnel-shaped subcutaneous conduit 100 to the subcutaneous tissues 210.

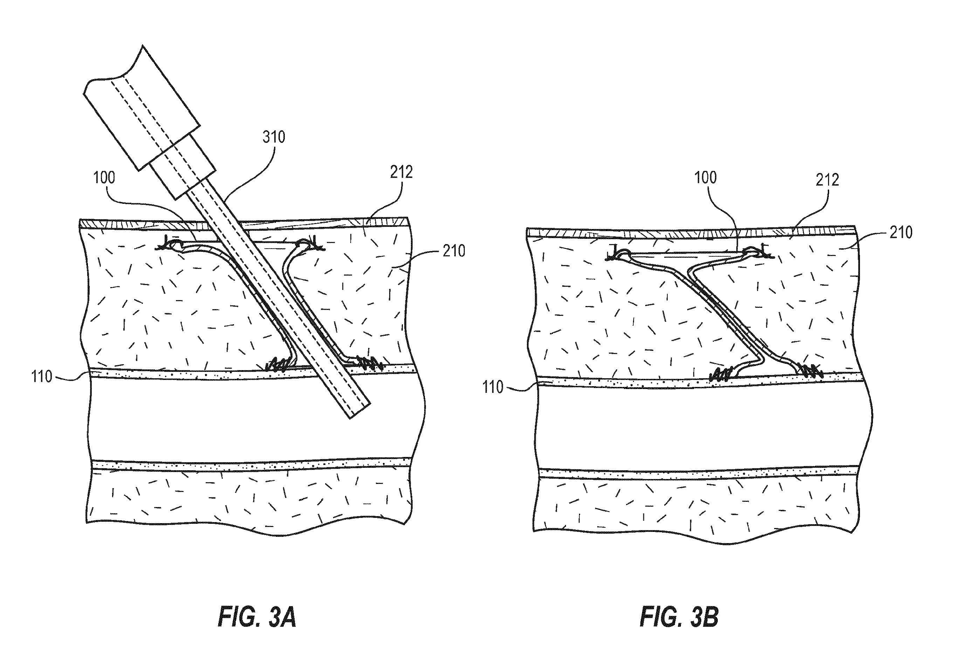

In certain embodiments, the funnel-shaped subcutaneous conduit 100 may be configured for coaptation (reversible collapse) of the walls of the funnel-shaped subcutaneous conduit 100, when not separated by a needle or catheter, to reduce or eliminate central dead space within the funnel-shaped subcutaneous conduit 100. For example, FIG. 3A is a cross-section view of a sheathed needle 310 accessing the blood vessel 110 through the funnel-shaped subcutaneous conduit 100 according to one embodiment. Advancing the sheathed needle 310 through the funnel-shaped subcutaneous conduit 100 forces separation between the walls of the funnel-shaped subcutaneous conduit 100. When the sheathed needle 310 is removed, however, the lack of a minor opening force on the walls allows the funnel-shaped subcutaneous conduit 100 to coapt. FIG. 3B is a cross-section of the funnel-shaped subcutaneous conduit 100 in a coapted state according to one embodiment.

In some embodiments, an optional supporting structure (not shown) of the funnel-shaped subcutaneous conduit 100 may, for example, be provided by a stent-like structure composed of a material such as Nitinol^{®}, Conichrome^{®} (or other chromium-nickel-molybdenum-iron alloy specified by ASTM F1058 or ISO 5832-7), or other elastic or superelastic material. As discussed above, the stent may keep the walls of the body of the funnel-shaped subcutaneous conduit 100 coapted to reduce or obliterate dead space within the needle conduit when not engaged by a needle or catheter. In other embodiments, the elastic or superelastic material may be used to facilitate insertion of the funnel-shaped subcutaneous conduit 100 through minimally invasive surgical tools. For example, the funnel-shaped subcutaneous conduit 100 may be compressed and retained by a sheath during insertion. After insertion, the restraining sheath may be removed to allow the funnel-shaped subcutaneous conduit 100 to expand.

In certain embodiments, magnetic elements or wires (not shown) may also be incorporated into the funnel-shaped subcutaneous conduit 100 to help guide a needle through the funnel-shaped subcutaneous conduit 100 by magnetic deflection. Alternatively, a wire structure (such as a cone composed of woven wires) (not shown) that displays magnetism may be used so that a needle may be guided to a correct vessel puncture point by magnetic deflection. In other words, the magnetic deflection of the wire structure keeps the needle on course toward the correct puncture point. The wire structure may be located in the subcutaneous tissues and attached to the adventitia of a target blood vessel, similar to the funnel-shaped subcutaneous conduit 100 discussed above. The magnetic elements in the guide (and as necessary in the needle) can be as those described in U.S. Patent No. 7,059,368 issued to Filler (the '368 patent), which is hereby incorporated by reference herein in its entirety. Care is taken to select materials suitable for integration in a mammalian body, as opposed to materials use with vials and the like contemplated in the '368 patent. In magnetically guided embodiments, the armoring can be reduced or dropped, as the magnetic feature guides the needle through the funnel-shaped subcutaneous conduit 100. The needle guide may be substantially a metallic wire frame.

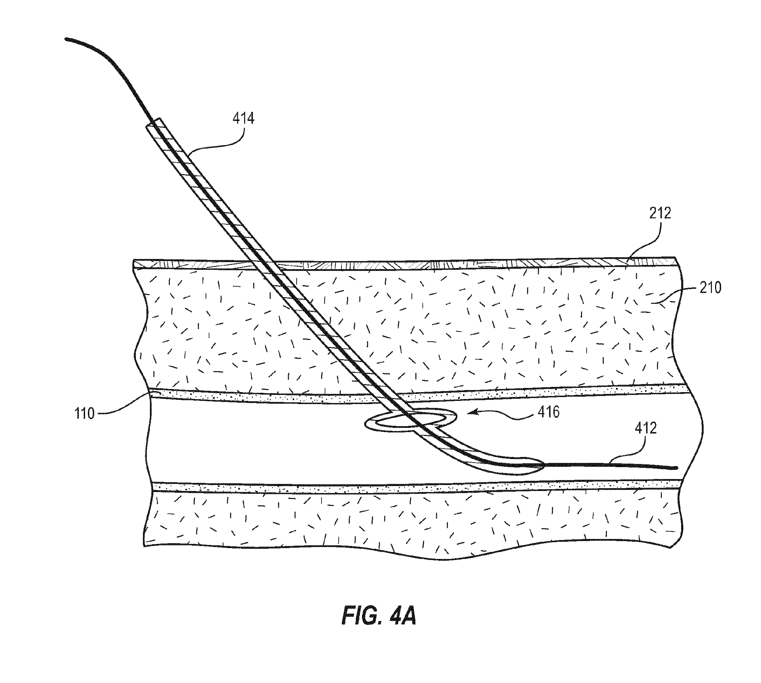

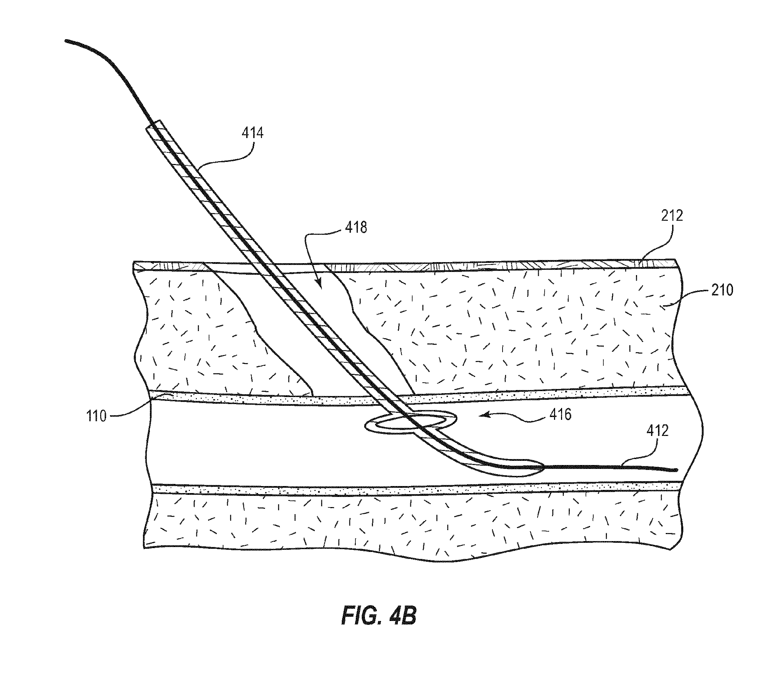

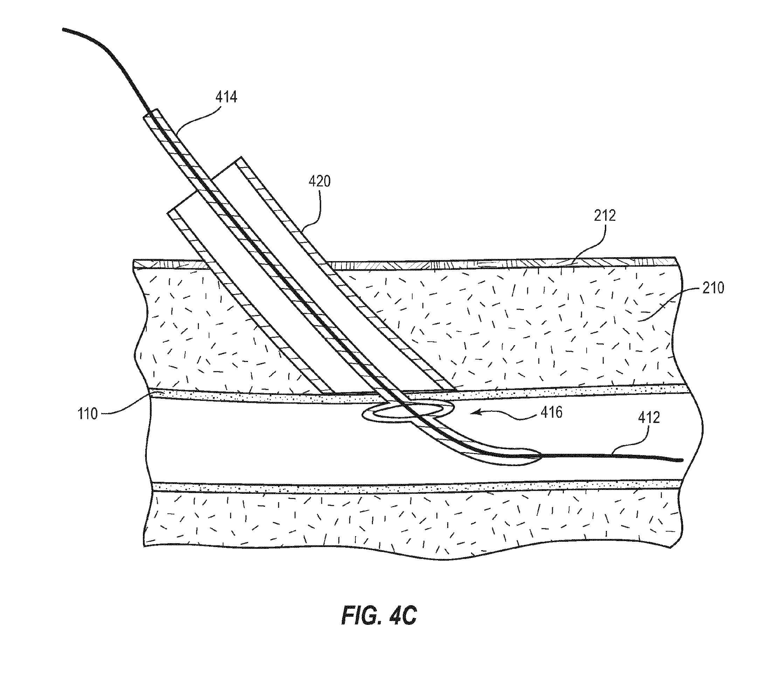

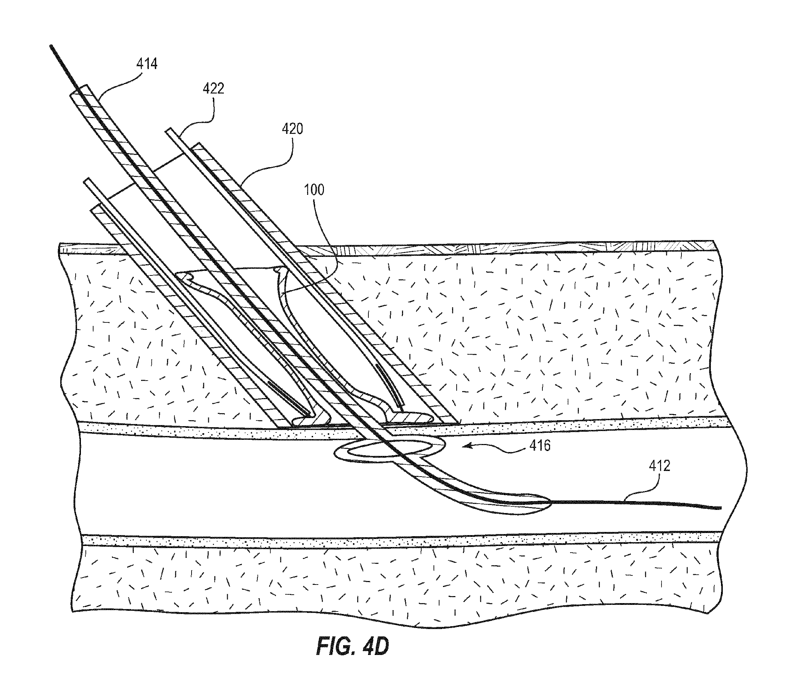

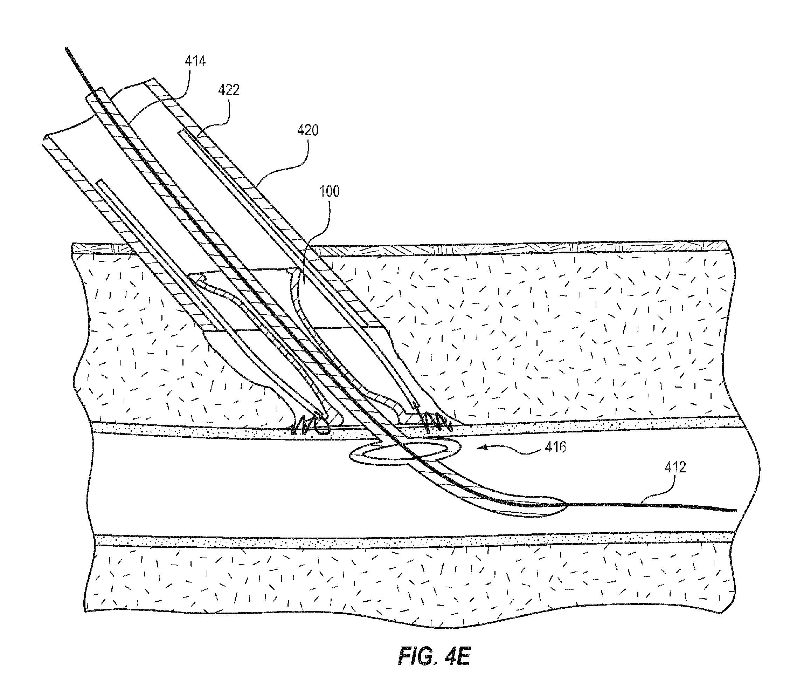

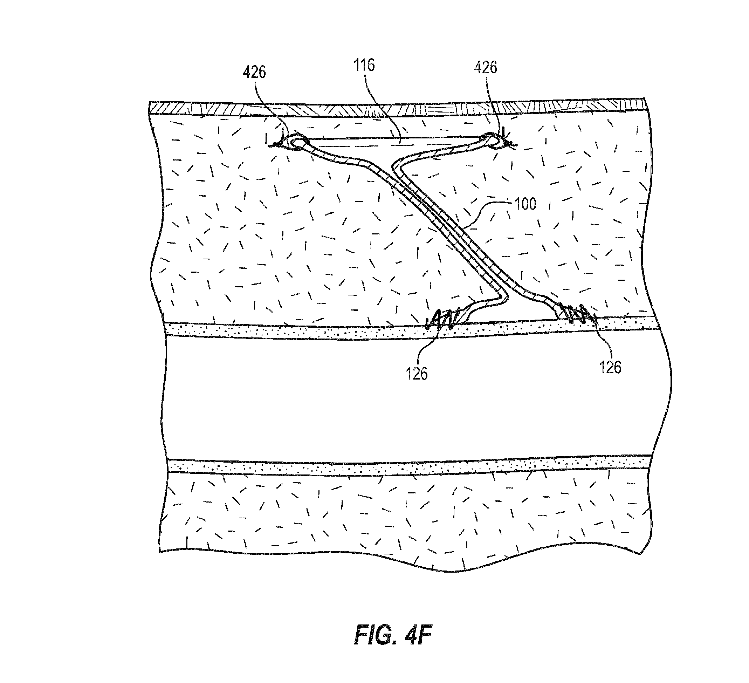

FIGS. 4A, 4B, 4C, 4D, 4E, and 4F are cross-section views illustrating a method for inserting the funnel-shaped subcutaneous conduit 100 into a patient according to one embodiment.

Referring to FIG. 4A, the blood vessel 110 (e.g., any target vein or artery) may first be punctured with a micropuncture (not shown) (e.g., a 21 gauge needle + 0.018" wire + 4-5 F entry sheath) system. This allows placement of a guidewire 412 *(e.g.,* a stiff 0.035" guidewire) over which a dilator 414 (*e.g.,* 6-8 F) with a retaining segment 416 (e.g., a Malecot segment with two to four "wings") may be advanced into the blood vessel 110. The retaining segment 416 may be extended/deployed such that when the dilator 414 is pulled back it is stopped at the vessel entry point by the retaining structure 416.

Referring to FIG. 4B, the skin entry zone of the dilator 414 may be incised with a scalpel and a subcutaneous pouch 418 for the funnel-shaped subcutaneous conduit 100 may be bluntly dissected.

Referring to FIG. 4C, over the dilator 414, a sheath 420 (e.g., a 10-12 F beveled tip sheath) with its dilator (not shown) may be advanced to the adventitial surface of the target blood vessel 110. The sheath's dilator may then be removed.

Referring to FIG. 4D, through the sheath 420, the operator may advance the funnel-shaped subcutaneous conduit 100 (which may be in a compressed state). As discussed above, the distal end 114 end of the funnel-shaped subcutaneous conduit 100 may include tabs 120 (e.g., two or more) adapted to facilitate attachment of the distal end 114 to the adventitial layer of the blood vessel 110. Hypotubes 422, one attached to each tab 120, may be advanced down the sheath 420 with the funnel-shaped subcutaneous conduit 100. Each hypotube 422 includes therein a minute "curly-Q" or helically shaped length of wire (referred to herein as helical wire 126) (e.g., of superelastic Nitinol^{®} or other suitable material) with a sharp distal end. A portion of each helical wire 126 is adapted to attach to a respective tab 120 at the distal end 114 of the funnel-shaped subcutaneous conduit 100.

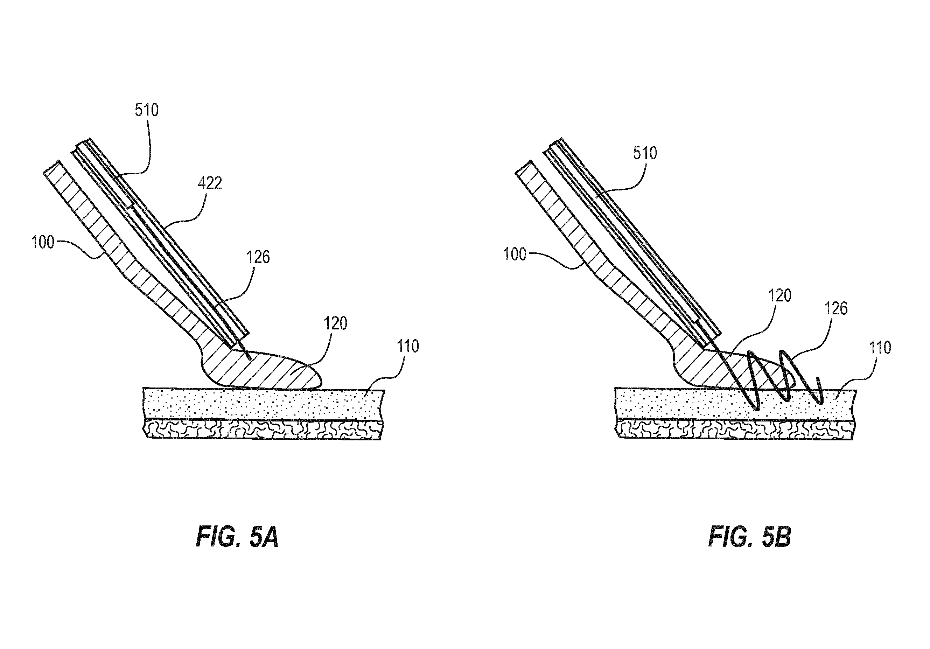

For example, FIGS. 5A and 5B are enlarged cross-section views of a hypotube 422 positioned adjacent to a tab 120 of the funnel-shaped subcutaneous conduit 100 according to one embodiment. As shown in FIG. 5A, while in the hypotube 22, the helical wire 126 may be in a straight configuration. In one embodiment, the helical wire 126 comprises superelastic Nitinol^{®} that is restrained by the hypotube 422 so as to be in the straight configuration. As shown in FIG. 5A, the sharp distal end of the helical wire 126 may extend from the distal end of the hypotube 422 so as to attach to the tab 120. The operator may then depress a proximal plunger 510 to force the helical wire 126 from the distal end of the hypotube 422. As shown in FIG. 5B, when the wire 126 is advanced out of the distal end of the hypotube by depressing the proximal plunger 510 on the hypotube 422, the superelastic wire 126 begins to reassume its predetermined helical shape. As the helical wire 126 is advanced out of the hypotube 422, the sharp distal end of the of the helical wire 126 repeatedly drives the helical wire 126 in and back out of the tab 120 and the adventitial layer of the blood vessel 110.

Referring to FIG. 4E, the operator knows when the hypotubes 422 are in the correct location for ligating wire deployment by advancing the hypotubes 422 within the sheath 420 until resistance is felt against the retaining segments 416 within the vessel lumen. As illustrated, before deploying the ligating mechanism (e.g., before depressing the proximal plungers 210 to force the helical wires 126 out the distal ends of the hypotubes 422), the sheath 420 may be withdrawn slightly as needed to facilitate the ligating mechanism. After wire deployment (which attaches the distal conduit tabs 120 to the external surface of the blood vessel 110), the hypotubes 422 can be removed. A pusher device (not shown), located against the collapsed proximal ring structure, is held stationary while the sheath 21 and hypotubes 422 are withdrawn, leaving the funnel-shaped subcutaneous conduit 100 (in a collapsed state in the illustrated embodiment) within the perivascular and subcutaneous tissues 210. Any exposed portion of the proximal end 112 of the funnel-shaped subcutaneous conduit 100 can be "tucked" into the subcutaneous tissue pouch 418. In certain embodiments, one or several minute eyelets (not shown) may be present along the periphery of the proximal ring 116 or at other locations on the funnel-shaped subcutaneous conduit 100, allowing the funnel-shaped subcutaneous conduit 100 to be sutured to the subcutaneous tissues 210 within the pouch 418. The retaining segment 416 of the dilator 414 may be retracted or collapsed, and the dilator 414 removed. Direct manual pressure may be applied to the entry site for several minutes to ensure hemostasis. Finally, the entry site incision may be repaired with subcuticular sutures (not shown). The funnel-shaped subcutaneous conduit 100 should be ready for use within ten to fourteen days.

FIG. 4F shows the funnel-shaped subcutaneous conduit 100 with sutures 426 securing the proximal ring 116 to the subcutaneous tissue 210. The entry site incision is shown as repaired and the funnel-shaped subcutaneous conduit 100 is shown as ready for use. The funnel-shaped subcutaneous conduit 100 may be used for repetitive entry into both veins and arteries. However, the funnel-shaped subcutaneous conduit 100 may also be useful for repetitive entry into other bodily cavities, such as the cerebrospinal fluid (CSF) space, the biliary tree, the urinary system, etc. The funnel-shaped subcutaneous conduit 100 may also serve as a pathway to allow rapid reentry for deep body access for biopsies, fluid drainage, laparoscopy, etc. The funnel-shaped subcutaneous conduit 100 may also allow repetitive access to the inferior vena cava and the abdominal aorta via the trans lumbar route. In these cases, initial access of the target vessel or other structures may, for example, be accomplished under computed tomography (CT) scan guidance (or other imaging guidance) with skinny needle puncture. The structures against which the funnel-shaped subcutaneous conduit 100 may be affixed to facilitate periodic piercing can be termed "biological boundary structures."

The above description of implanting the funnel-shaped subcutaneous conduit 100 within a patient has been described with regards to an embodiment wherein the funnel-shaped subcutaneous conduit 100 remains coapted until a sheathed needle is inserted therethrough to access the blood vessel 110. In other embodiments, however, the funnel-shaped subcutaneous conduit 100 may be inserted in a collapsed state, but may then be expanded in place to create an open passageway to the blood vessel 110. For example, the sheath 420 may be configured to restrain the funnel-shaped subcutaneous conduit 100 in a collapsed state during insertion, and removal of the sheath 420 allows the funnel-shaped subcutaneous conduit 100 to expand within the perivascular and subcutaneous tissues 210. In another embodiment, a balloon (not shown) may be inflated to expand the funnel-shaped subcutaneous conduit 100 after insertion.

An artisan will recognize from the disclosure herein many alternatives for implanting the funnel-shaped subcutaneous conduit 100 within a patient. For example, a system for remotely ligating the funnel-shaped subcutaneous conduit 100 to the adventitial layer of the vessel 110 may be similar to the remote ligation system marketed as the Q-wire by the Davol division of C.R. Bard Inc. Other remote ligation systems that may be used with the funnel-shaped subcutaneous conduit 100 include crimping of a metallic or resorbable surgical clip, which may be remotely engaged with the blood vessel 110 for example by pulling back on a plunger in the deploying device. A resorbable or nonresorbable surgical suture may also be used to affix the funnel-shaped subcutaneous conduit 100 to the target vessel 110.

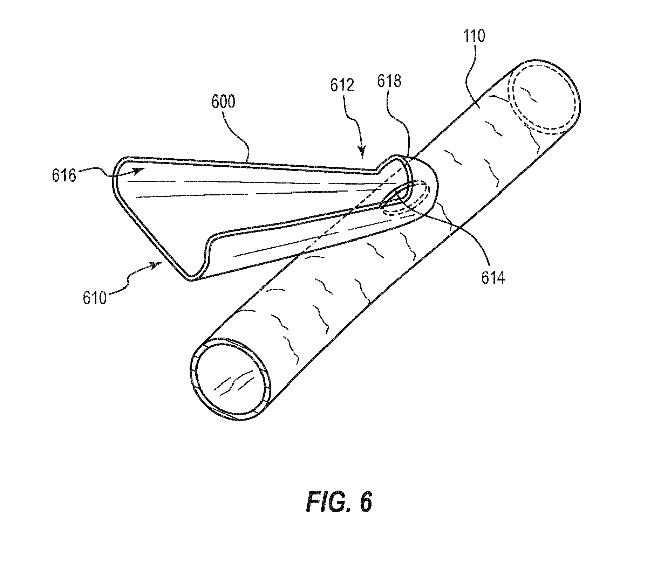

Further, needle conduits having other shapes (rather than the illustrated funnel shape) may also be used. For example, FIG. 6 is a perspective view of a sluice-shaped or "dust pan" subcutaneous conduit 600 according to one embodiment. The sluice-shaped subcutaneous conduit 600 is shown situated on a blood vessel 110. The sluice-shaped subcutaneous conduit 600 includes a first end 610 that is wider than a second end 612. The wider first end 610 serves to locate a needle entry point and provides guidance for directing the needle to the second end 612. The second end 612 includes an eyelet 614 that is shown positioned over an intended venous entry point. The sluice-shaped subcutaneous conduit 600 includes folded over lips 616 at the edges of the sluice-shaped subcutaneous conduit 600 that help direct a needle towards the eyelet 614 affixed to the blood vessel 110. As shown, the sluice-shaped subcutaneous conduit 600 may include a hood 618 over the eyelet 614 to prevent the needle from sliding beyond the intended venous entry point and to further direct the needle to the eyelet 614. Although not shown, second end 612 of the sluice-shaped subcutaneous conduit 600 may include tabs for suturing the second end 612 to the adventitial layer of the blood vessel 110.

Other systems and methods may also be used to implant a subcutaneous conduit within a patient. For example, in one embodiment, two guidewires are used. In such an embodiment, a first guidewire is advanced through a first incision directly into a target vessel. A second guidewire is inserted through a second incision and tunneled under the skin to the first incision location similar to, for example, the tunneling of a Hickman catheter. The second guidewire is used for insertion of the subcutaneous conduit. In some embodiments, the subcutaneous conduit may be in a collapsed state during insertion and may be expanded in place by, for example, removing a sheath or expanding a balloon. Once the subcutaneous conduit is in place, the first guidewire is used for affixing the eyelet of the subcutaneous conduit directly to a desired location of the target vessel. For example, the first guidewire may be used with a suture device (such as a Perclose^{®} suture device or other mechanical closure device) to precisely place sutures at or near the eyelet or distal opening of the subcutaneous conduit. Both guidewires may then be removed and both incisions repaired. The subcutaneous conduit may then be used after healing for ten to fourteen days.

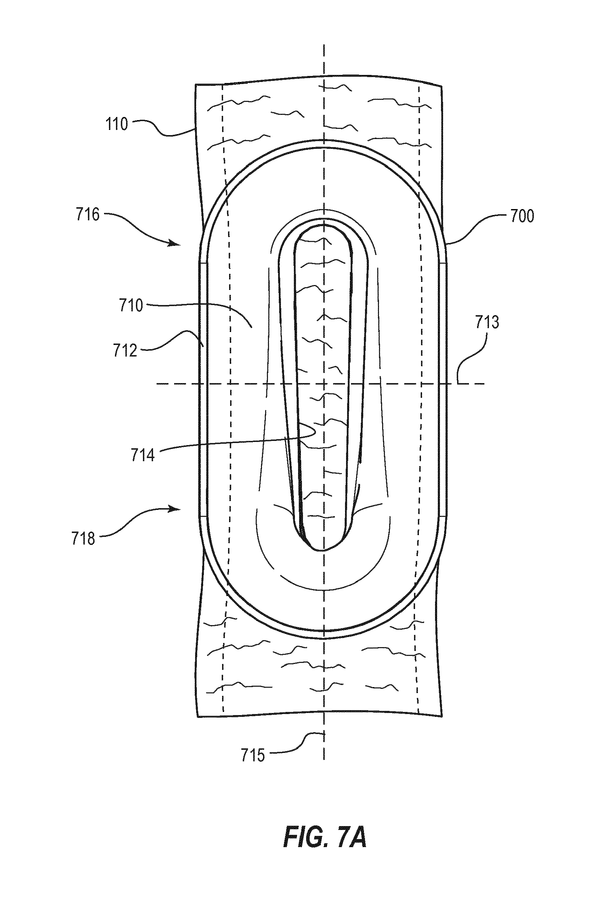

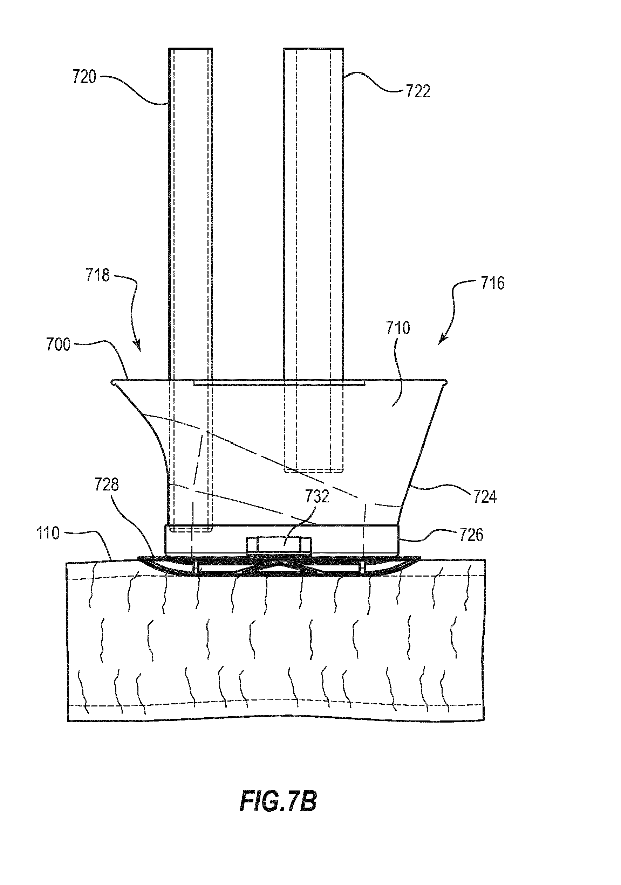

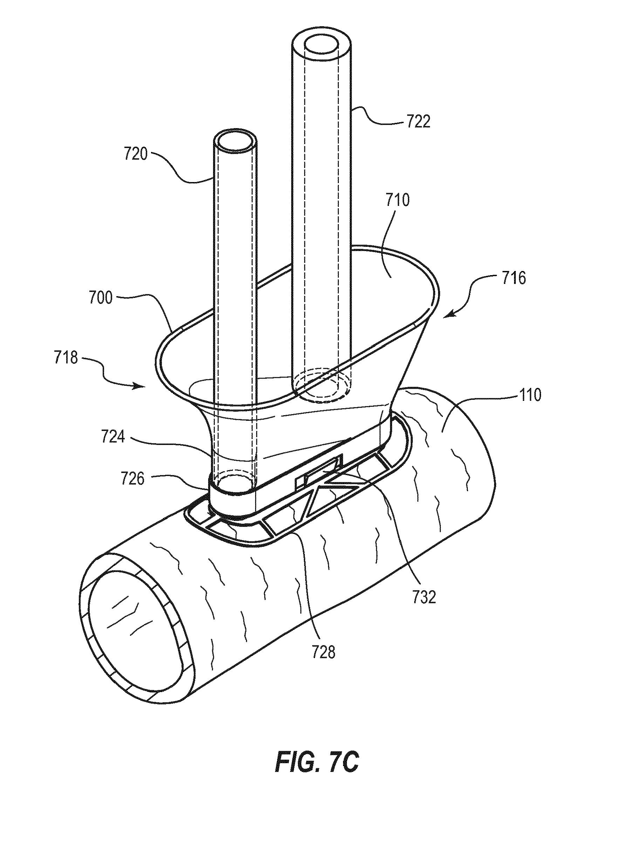

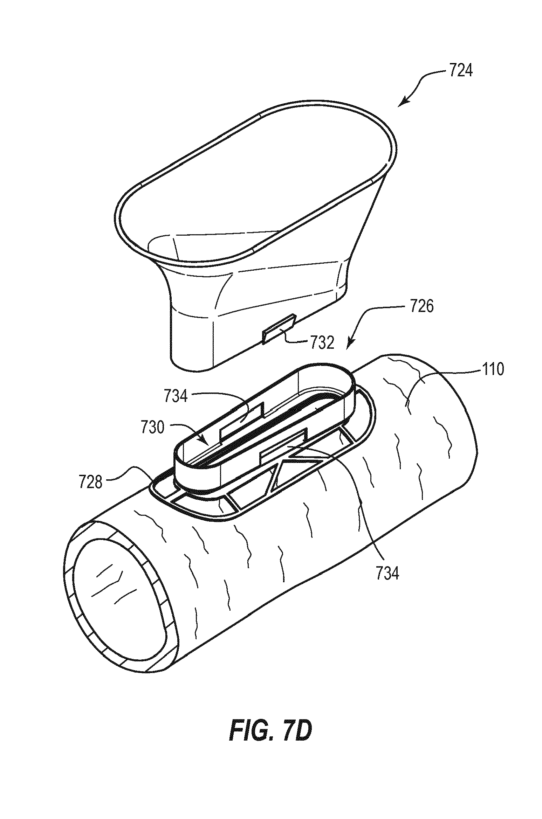

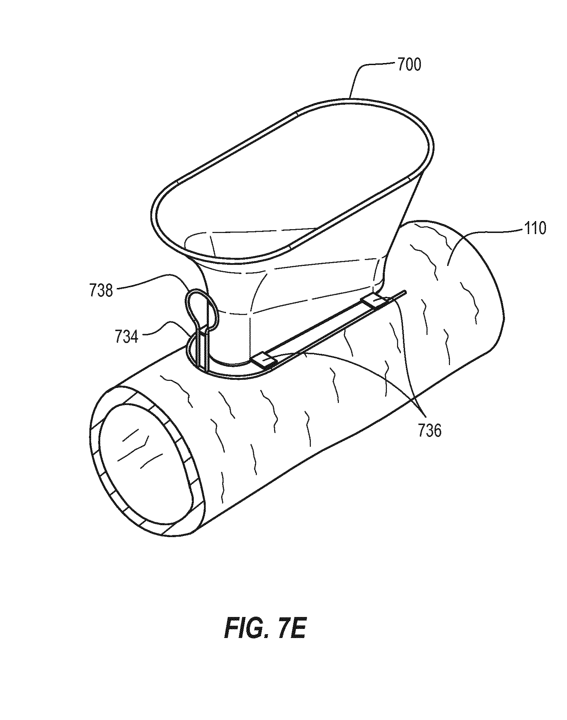

FIGS. 7A, 7B, 7C, 7D, and 7E schematically illustrate an elongated funnel-shaped subcutaneous conduit 700 according to certain embodiments. The elongated funnel-shaped subcutaneous conduit 700 is shown with respect to a blood vessel 110. Repeated access to the same site can result in damage to the blood vessel 110. Thus, a user may access different locations along the blood vessel 110 through the elongated funnel-shaped subcutaneous conduit 700 by selecting different sizes of canulas. On one day, for example, a user may access the blood vessel 110 at one end of the elongated funnel-shaped subcutaneous conduit 700 by using a canula with a relatively small outer diameter. On another day, the user may access the blood vessel 110 at another end of the elongated funnel-shaped subcutaneous conduit 700 by using a canula with a relatively large outer diameter.

FIG. 7A illustrates a top view of the elongated funnel-shaped subcutaneous conduit 700, as attached to the blood vessel 110. The elongated funnel-shaped subcutaneous conduit 700 includes walls 710 that narrow from a proximal opening 712 to a distal opening 714. The proximal opening 712 and the distal opening 714 each have a short axis 713 and a long axis 715. The elongated funnel-shaped subcutaneous conduit 700 is implanted in a patient such that the distal opening 714 is adjacent the blood vessel 110 with the long axis 715 aligned with the axis of the blood vessel 110. Thus, after the oval funnel-shaped subcutaneous conduit 700 is implanted, a user locates the large proximal opening 712 and inserts a sheathed needle or other canula (see FIGS. 7B and 7C), which the walls 710 of the funnel-shaped subcutaneous conduit 700 directs to the blood vessel 110 through the distal opening 714.

The short axis 713 of the distal opening 714 is larger at a first end 716 than it is at a second end 718 of the elongated funnel-shaped subcutaneous conduit 700. In other words, the distal opening 714 tapers in size from the first end 716 to the second end 718. The taper directs a canula with a relatively larger outer diameter to the larger end 716 of the elongated funnel-shaped subcutaneous conduit 700, while allowing a canula with a smaller outer diameter to pass through the smaller end of the of the distal opening 714. The tapering allows a user to select one of several access points along the vessel 110 by selecting the diameter of the access canula.

FIG. 7B illustrates a side view of the elongated funnel-shaped subcutaneous conduit 700, as attached to the blood vessel 110. FIG. 7C illustrates a perspective view of the elongated funnel-shaped subcutaneous conduit 700, as attached to the blood vessel 110. For illustrative purposes, FIGS. 7B and 7C show a first canula 720 and a second canula 722 that a user may select for gaining access to the blood vessel 110. A user may, for example, decide to use the first canula 720 at a first time to access the blood vessel 110 at a first site and the second canula 722 at a second time to access the blood vessel 110 at a second site. As shown, the first canula 720 has an outer diameter that is smaller than the outer diameter of the second canula 722. Thus, the first canula 720 may access the blood vessel at the narrower or second end 718 of the elongated funnel-shaped subcutaneous conduit 700, whereas second canula 722 is sufficiently large such that it can access the blood vessel 110 at only the wider or first end 716 of the elongated funnel-shaped subcutaneous conduit 700. Although not shown, additional canulas having different outer diameters may be used to access different sites along the length of the elongated funnel-shaped subcutaneous conduit 700 at desired points between the first end 716 and the second end 718. The sides 710 of the elongated funnel-shaped subcutaneous conduit 700 may also be sloped towards the larger end 716 of the distal opening 714 (shown in FIG. 7A) to further direct larger canula 722 to the larger end 716 of the elongated funnel-shaped subcutaneous conduit 700.

The embodiment shown in FIGS. 7B, 7C, and 7D is a two-piece design that includes a funnel portion 724 and a base portion 726. FIG. 7D is a perspective view illustrating the funnel portion 724 separated from the base portion 726. In the illustrated embodiment, the base portion 726 includes a curved, flexible lattice 728 configured for suturing to the wall (e.g., the adventitial layer) of the blood vessel 110. For illustrative purposes, the drawings do not show the sutures securing the base portion 726 to the blood vessel 110. As shown in FIG. 7D, the base portion may include a pocket 730 for receiving the funnel portion 724. The funnel portion 724 may snap into the base portion 726. For example, as illustrated, the funnel portion 724 may include two retaining tabs 732 (one on either side) protruding from the funnel portion 724 that fit into matching openings 734 in the base portion 726. Squeezing the sides of the funnel portion 724 releases the retaining tabs 732 from the openings 734 to separate the funnel portion 724 from the base portion 726. The two-piece design provides easier access to the base portion 726 during the implant and explant procedures. For example, a user may implant the elongated funnel-shaped subcutaneous conduit 700 by excising a pocket of subcutaneous tissue (not shown) sufficient to fit the elongated funnel-shaped subcutaneous conduit 700, suturing the base portion 726 to the adventitial layer of the blood vessel 110 (e.g., using a suturing system), and snapping the funnel portion 724 into place in the base portion 726. To explant the elongated funnel-shaped subcutaneous conduit 700, a user may squeeze the sides of the funnel portion 724 to release the funnel portion 724 from the base portion 726 so that the sutures may be cut and removed.

FIG. 7E is a perspective view of a one-piece funnel design (e.g., that does not include the base portion 726 illustrated in FIGS. 7B, 7C, and 7D) of the elongated funnel-shaped subcutaneous conduit 700, as attached to the blood vessel 110. The embodiment of the elongated funnel-shaped subcutaneous conduit 700 shown in FIG. 7E may be attached to a suture wire 734 through a plurality of tabs 736 (two shown). The suture wire 734 may be located along both sides of the elongated funnel-shaped subcutaneous conduit 700. Sutures (not shown) may be used to secure the suture wire 734 to the adventitial layer of the blood vessel 110. Thus, the attached elongated funnel-shaped subcutaneous conduit 700 is secured to the blood vessel 110. The suture wire 734 may include a central tab 738 that may be pulled like a rip-cord to release the elongated funnel-shaped subcutaneous conduit 700 from the blood vessel 110. When the central tab 738 is pulled, the suture wire 734 separates from the tabs 736 such that the suture wire 734 may slip free from the sutures, which also allows the elongated funnel-shaped subcutaneous conduit 700 to be removed from the patient. Thus, a user may easily access and remove the sutures.

The embodiments discussed below are directed to detection and guide systems for accessing blood vessels. An implant formed of a biocompatible material is implanted within a mammalian body proximate an outer surface of a biological boundary structure, such as a blood vessel. The detection and guide system may be configured to be positioned outside the mammalian body to detect the implant and to guide a needle to the desired entry site of the biological boundary structure. A needle guide may be coupled to a housing of the detection and guide system and directed at an angle such that positioning the implant detector over the implant aligns an opening through the needle guide with the desired entry site. As discussed in detail below, one embodiment of a detection and guidance system provides metal detection of a metallic implant. Other example embodiments described below provide magnetic detection of a magnetic implant.

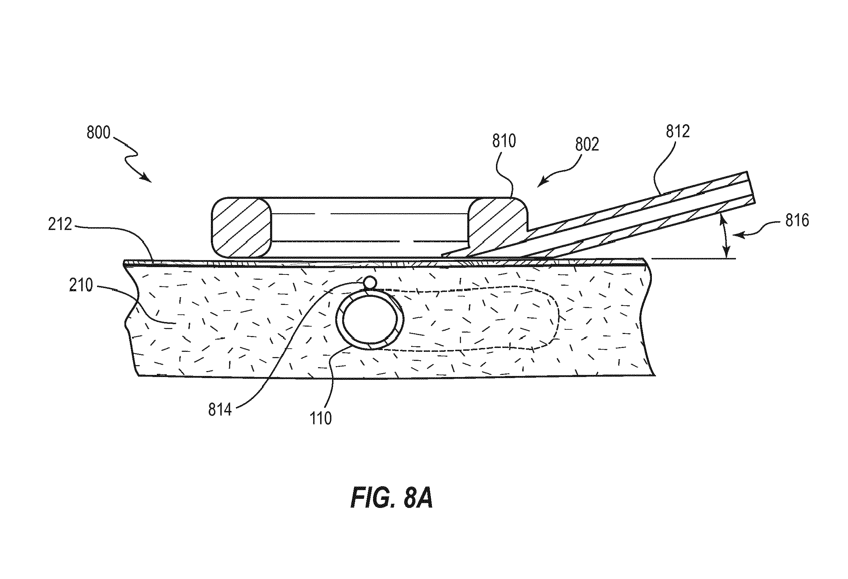

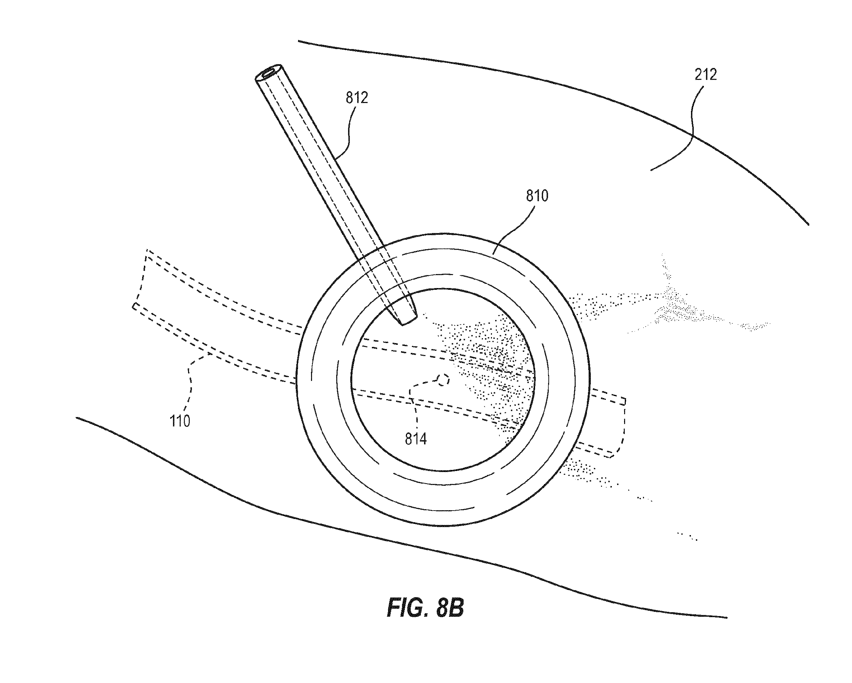

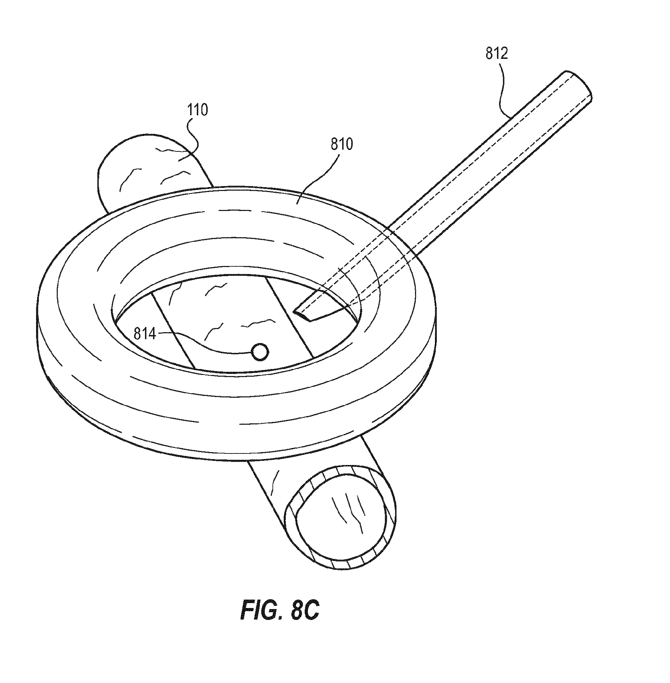

FIGS. 8A, 8B, and 8C schematically illustrate a system 800 for accessing a biological boundary structure, such as a blood vessel 110, at a desired entry site according to one embodiment. FIG. 8A is a cross-section side view of the system 800 in use external to a patient's skin 212 to locate a desired entry site on the blood vessel 110. The blood vessel 110 is located in subcutaneous tissue 210. FIG. 8B is a top view of the system 800 in use. FIG. 8C is a perspective view of the system 800 in use (with the skin 212 and subcutaneous tissue 210 omitted for illustrative purposes). The illustrated system 100 includes an implant 814 and a detection and guide system 802. The implant 814 may be formed of a biocompatible material and may be configured to be implanted adjacent the blood vessel 110.

The system 800 illustrated in FIGS. 8A-8C may be a metal detection system configured to detect a metallic implant 814. The detection and guide system 802 of the metal detection system 800 includes one or more metal detector coils (not shown) within a coil housing 810. A needle guide, such as a guide canula 812, may be attached to the coil housing 810. The metal detection system 800 also includes a metallic implant 814. Although not shown, the metal detection system 800 also includes an oscillator for producing an alternating current that passes through the one or more coils to produce an alternating magnetic field. When the coil housing 810 is sufficiently close to the metallic implant 814, the alternating magnetic field generated by the magnetic coils produce eddy currents in the metallic implant 814 such that the metallic implant 814 produces another alternating magnetic field. The one or more coils are then used to detect the alternating magnetic field produced by the metallic implant 814. The metal detection system 800 may provide audio and/or visual indicia of metal detection.

The metal detection system 800 may be used to detect the metallic implant 814. The guide canula 812 may be rigidly fixed to the coil housing 810 to provide accurate guidance of an access needle to the desired access site near the detected metallic implant 814.

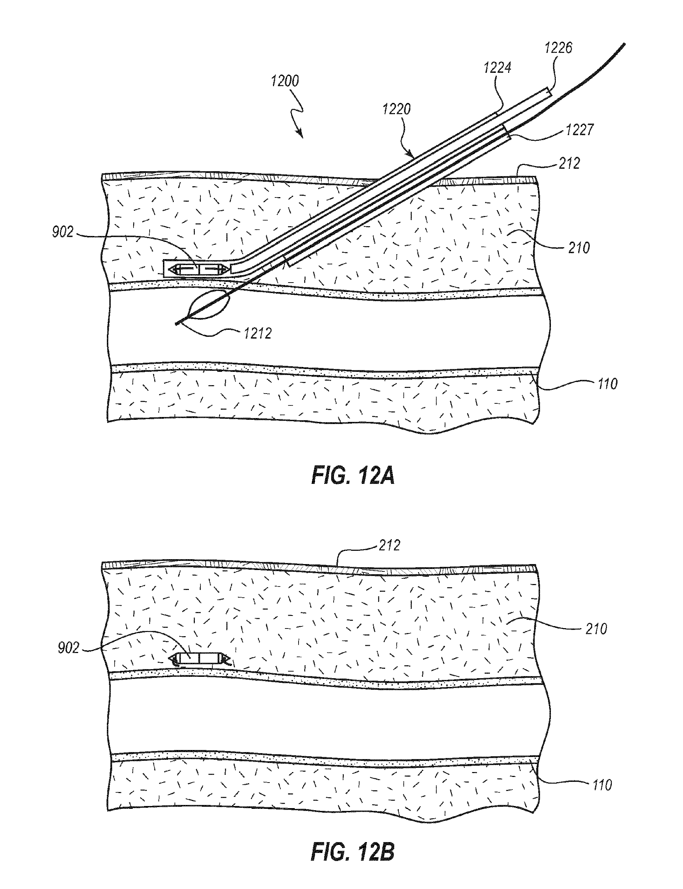

In one embodiment, the metallic implant 814 comprises a small spherical-shaped piece of metal suitable for human implant, such as stainless steel or titanium. The metallic implant 814 may be placed on or above the targeted blood vessel 110, for example, using open surgery or with a low invasive procedure through a small hypodermic needle. A system and method of inserting an implant is illustrated in FIG. 12A and discussed below with reference to the same.

As can be appreciated, the metallic implant 814 may simply include a portion of metallic material and need not be formed entirely of metal. For example, the implant 814 may comprise a biocompatible plastic shell surrounding a piece of metal.

In certain embodiments, the metallic implant 814 may be part of, or integrated with a subcutaneous conduit, such as the subcutaneous conduit embodiments described herein. Given fixed properties (e.g., material and mass) of the metallic implant 814, the metal detection system 800 may be calibrated to accurately find both the planar (e.g., in X and Y directions) position and the depth (e.g., in a Z direction) of the metallic implant 814. Thus, in certain embodiments, an angle 816 of the guide canula 812 with respect to a plane of the patient's skin 212 may be adjusted based on the detected depth of the metallic implant 814. In one such embodiment, the guide canula 812 may include a hinge structure (not shown) to allow the angle 816 to be adjusted based on the detected depth. In another embodiment, a user may select one of multiple fixed guide canulas 812 that each provide a different angle 816 based on the metal detector's depth reading.

In addition, or in other embodiments, a platform (not shown) may be used to steady the metal detector system 800 over the patient to increase accuracy.

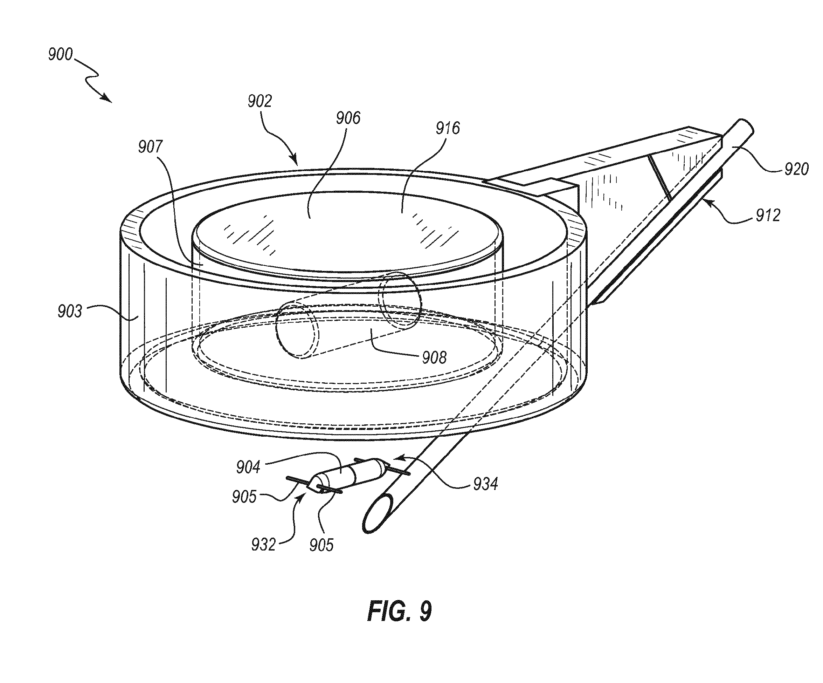

FIG. 9 is a perspective view of another system 900 for accessing a biological boundary structure, such as a blood vessel 110, at a desired entry site according to another embodiment. The system 900 includes a detection and guide system 902 and an implant 904. The detection and guide system 902 is configured to detect the implant 904 and guide a needle 920 to the desired entry site. The detection and guide system 902 includes an implant detector 906 positioned within a housing 903. A needle guide 912 may be attached to the housing 903. A needle 920 is shown positioned in the needle guide 912.

The implant 904 includes a biocompatible material and is configured to be implanted into a mammalian body. In the illustrated embodiment, the implant 904 has a capsule-like shape with pointed ends. The implant 904 includes a magnetic material that creates a magnetic field that can be detected outside of a body in which the implant is implanted. A first end 932 of the implant 904 may be configured as a north pole and a second end 934 of the implant 904 may be configured as a south pole. The implant 904 may also include barbs 905 configured to affix the implant 904 within subcutaneous tissue and/or adventitia of the vessel 110 within the mammalian body into which the implant 904 is implanted. The barbs 905 may be formed of a superelastic Nitinol^{®} (NiTi) configured to collapse into an implantation position during an implant procedure and spring to a securement position once the implant is properly positioned. The implant 904 is shown in more detail in FIG. 12, and described below in greater detail with reference to the same.

The implant detector 906 may be configured to detect a detectable material of the implant 904, such as a metal or a magnetic material. In the illustrated embodiment, the implant detector 906 is a compass having a housing 907 and a locator 908. In the illustrated embodiment, the compass 906, and more specifically the locator 908 (e.g., a magnet), may be configured to detect the magnetic field produced by the magnetic material of the implant 904. The housing 907 of the compass 906 may be shaped as a shallow cylinder having a sidewall, a floor at one end and an opening opposite the floor. A transparent cover 916 may be positioned on the top of the housing 907, over the opening, to allow visibility of the locator 908 within the housing 907.

The locator 908 may be positioned within the housing 907 of the implant detector 906 (e.g., compass) and may be configured to be responsive to the magnetic field of the implant 904 when the locator 908 is positioned sufficiently within the magnetic field of the implant 904. In the illustrated embodiment, the locator 908 may be configured to be able to shift position relative to the housing 907 of the implant detector 906 and the needle guide 912 and a housing 903 of the detection and guide system 902.

Although not shown, in certain embodiments, the locator 908 is configured to rotate around a rod or spindle. In the embodiment illustrated in FIG. 9, however, the locator 908 is free floating in a liquid (e.g., water) within the housing 907 of the implant detector 906. As the implant detector 906 is moved near the implant 904, and into the magnetic field of the magnetic material of the implant 904, the locator 908 may move within the housing 907 relative to the approaching magnetic field. Further movement of the implant detector 902 relative to the magnetic field of the implant 904 may cause the locator 908 to gradually shift position within the housing 907, in response to the shifting magnetic field. The locator 908 may have a tendency to move toward a position closest to the magnetic field that is accessible to the locator 908. The position closest to the magnetic field will be closest to the implant 904 and will be, as nearly as possible, directly above the implant 904. Stated differently, the locator 908 may tend to move toward a position that is outside the mammalian body on a line from the implant 904 that is orthogonal to a plane of the outer surface of the mammalian body. Accordingly, when the locator 908 is positioned directly in the center of the housing 907, the implant detector 906 is properly positioned over the implant 904.

In the illustrated embodiment, the locator 908 may be configured to also align with the poles of the magnetic material of the implant 904. The poles of the magnetic material of the implant 904 may be configured according to a desired magnetic field direction. The direction of the magnetic field of the implant 904 can then be used to align the orientation of the needle guide 912 relative to the implant 904 to determine the desired entry site (or re-entry site).

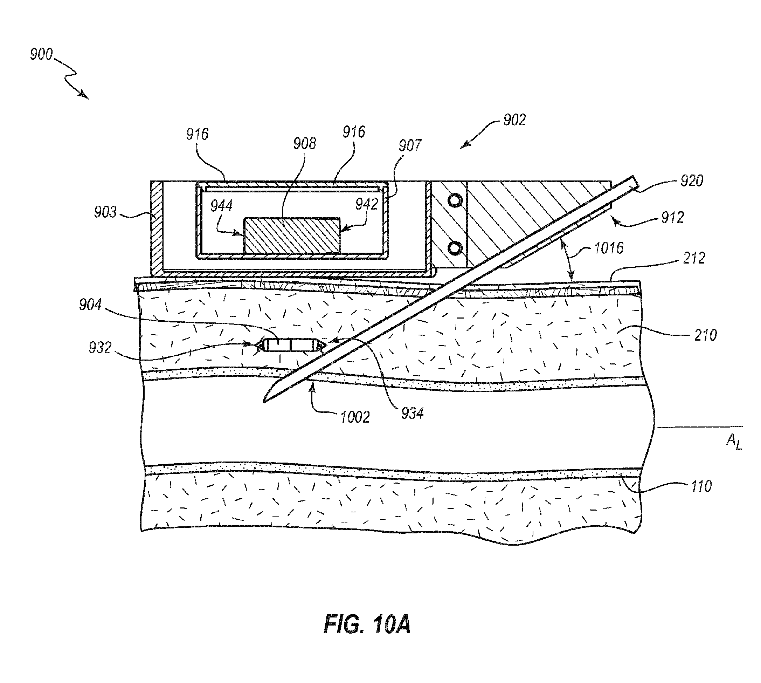

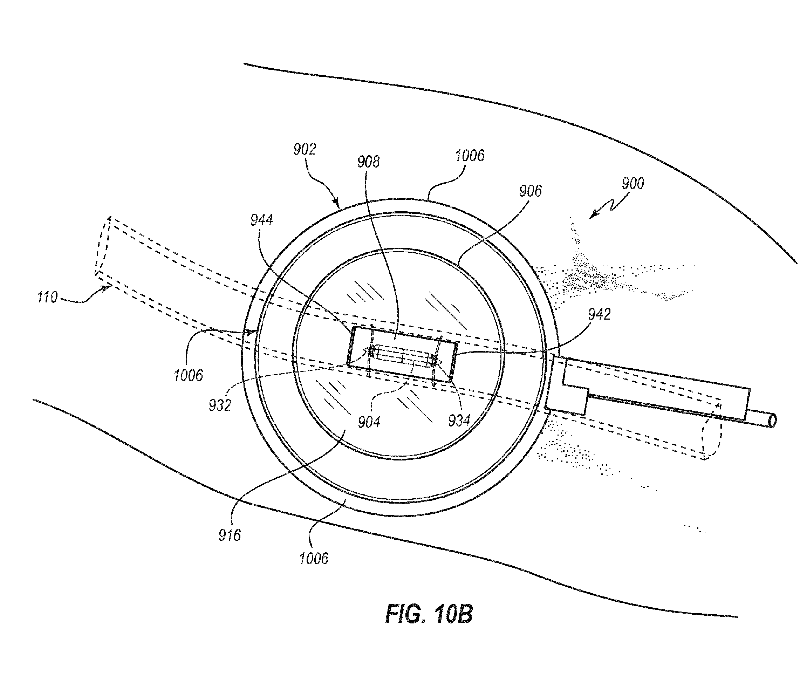

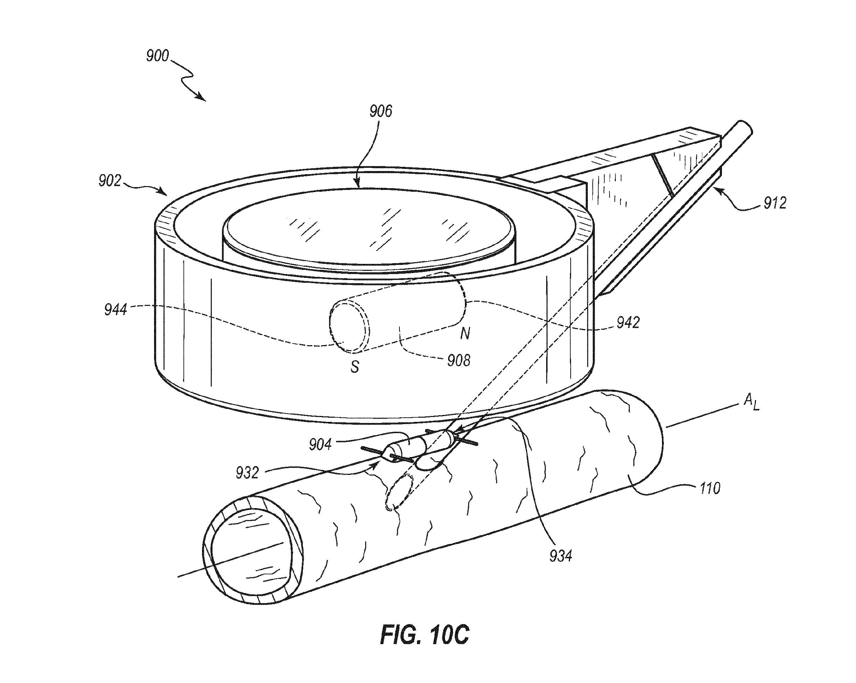

FIGS. 10A-10C are side, top, and perspective views, respectively, of the system 900 for accessing a biological boundary structure, such as a blood vessel 110, at a desired entry site 1002. FIG. 10A is a cross-section side view of the system 900 in use external to a patient's skin 212 to locate a desired entry site on the blood vessel 110. FIG. 10B is a top view of the system 900 in use. FIG. 10C is a perspective view of the system 900 in use (with the skin 212 and subcutaneous tissue 210 omitted for illustrative purposes).

In FIGS. 10A-10C, the system 900 is aligned with a longitudinal axis A_{L} (shown in FIGS. 10A and 10C) of the blood vessel 110. More specifically, the needle guide 912 is aligned with the longitudinal axis A_{L} of the blood vessel 110 such that the needle 920 is entering the blood vessel 110 angled toward a direction that is in line with, or parallel to, the longitudinal axis A_{L}. The alignment of the needle guide 912 is accomplished because the first end 932 of the implant 904 (corresponding to the north pole of the magnetic material) and the second end 934 of the implant 904 (corresponding to the south pole of the magnetic material) are oriented parallel to the longitudinal axis A_{L} of the blood vessel 110. Thus, the magnetic field that is created by the implant 904 includes a component that is parallel with the longitudinal axis A_{L} of the blood vessel 110. The locator 908 in turn aligns with the direction of the magnetic field when positioned in the center of the implant detector 906.

In the illustrated embodiment, the locator 908 may be a cylindrical bar magnet, and thus configured to align with a magnetic field of the magnetic material of the implant 904. The locator 908 may have a north pole 942 and a south pole 944. When positioning the locator 908 near and/or over the implant 904, the magnetic fields of the locator 908 and implant 904 attract. The north pole 932 of the implant 904 attracts the south pole 944 of the locator and the south pole 934 of the implant 904 attracts the north pole 942 of the locator 902, as shown in FIG. 10B. When the locator 908 stabilizes substantially near the center of the housing 907 of the implant detector 906, a user can have a degree of certainty that the locator 908 indicates both the planar position (e.g., in X and Y directions in a plane of the surface of the patient's skin) and direction (or rotational orientation within the plane) of the implant 904.

The housing 907 of the implant detector 906 and/or the housing 903 of the detection and guide system 902 may include alignment markings 1006 to aid in aligning the needle guide 912 relative to the locator 908. As can be appreciated, when the locator 908 is aligned with the implant 904, indicating position and direction/orientation of the implant, the housing 907 of the implant detector 906 and/or the housing 903 of the detection and guide system 902 can be rotated about the locator 908. In this manner, the needle guide 912 can be brought into alignment with the implant. The markings can facilitate proper rotation (orientation) of the needle guide 912.

As can be appreciated, in other embodiments the locator 908 may be a thin piece of magnetic metal, similar to the needle of a compass. In certain embodiments, the locator 908 may be positioned in, or at least partially surrounded by, a liquid within the housing 907 of the implant detector 906. The liquid may facilitate alignment of the locator 908 with the magnetic field of an implant 904 by reducing frictional forces on the locator 908.

The needle guide 912 may be rigidly fixed to the housing 903 to provide accurate guidance of an access needle 920 to the desired access site 1002 near the detected implant 904. The needle guide 912 may be fixed at an angle 1016 (with respect to a plane of the patient's skin 212) directed toward a position below the center of the housing 903, as shown in FIG. 10A. In certain embodiments, the angle 1016 of the needle guide 912 may be adjustable. For example, the angle 1016 may be adjusted based on a detected depth of the implant 904. In one such embodiment, the needle guide 912 may include a hinge structure (not shown) to allow the angle 1016 to be adjusted based on the detected depth. In another embodiment, a user may select one of multiple fixed needle guides 912 that each provide a different angle 1016 based on the depth of the implant 904.

As illustrated in FIGS. 10A and 10C, the implant 904 may be implanted and/or affixed proximate an outer surface of a biological boundary structure, such as a blood vessel 110, of a mammalian body. The implant 904 may be positioned and/or affixed within subcutaneous tissue within the mammalian body. In another embodiment, the implant 904 may be affixed to adventitia of the blood vessel 110, or other biological boundary structure.

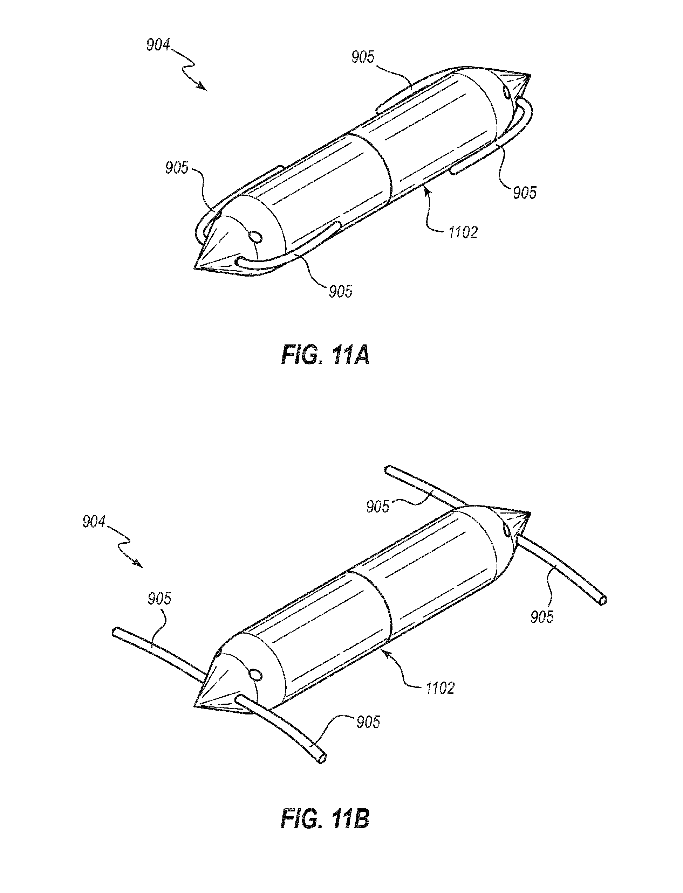

FIGS. 11A and 11B are perspecitve views of a detectable implant 904 of a system 900 for accessing a blood vessel at a desired entry site, according to one embodiment. In the illustrated embodiment, the implant 904 has a capsule shape (*i.e*., an elongate capped cylinder shape) having pointed ends. The elongate shape allows the implant 904 to have definite and disparate magnetic poles that can be easily aligned in a desired direction. A first end 932 of the implant 904 may be a magnetic north pole and a second end 934 of the implant 904 may be a magnetic south pole. The implant 904 may include a housing 1102 or outer shell to provide a desired shape and to contain a magnetic material, such as a bar magnet. The implant may also include barbs 905, as described below, which may affix or otherwise secure the implant 904 in a desired position within a mammalian body. The barbs 905 may include an elastic, biocompatible material, such as superelastic Nitinol^{®}.

FIG. 11A depicts the implant 904 with the barbs 905 in an implantation position. In the implantation position, the barbs 905 are folded toward the center of the implant to give the implant 904 a narrow profile. In another embodiment, the barbs are all folded in the same direction, for example in the insertion direction. The narrow profile of the implantation position allows for easy insertion into an implant delivery device and for easy implantation at a desired location within a mammalian body.

FIG. 11B depicts the implant 904 with the barbs 905 in an extended or securement position. The barbs 905 may be biased toward the securement position, such that when the barbs 905 are held in the implantation position and then released, the barbs 905 spring to the securement position. In the securement position, the barbs 905 may embed in surrounding tissue to thereby secure the implant 904 in place. The barbs 905 may limit longitudinal and/or rotational movement of the implant 904. While the barbs 905 of the illustrated embodiment are depicted as extending substantially straight, and orthogonal to a longitudinal axis of the implant 904, other configurations are possible. For example, the barbs 905 may have a slightly curved shape in the securement position, such that they may gently grip a biological boundary structure. In another embodiment, the barbs 905 may be configured to partially encircle a biological boundary structure, such as the blood vessel 110.

FIG. 12A illustrates a system 1200 and method for inserting a detectable implant 904, according to one embodiment The blood vessel 110 may first be punctured with a micropuncture (not shown) (e.g., a 21 gauge needle + 0.018" wire + 4-5 F entry sheath) system. This allows placement of a guidewire 1212 (e.g., a stiff 0.035" guidewire) with a retaining segment 1216. The retaining segment 1216 may be extended/deployed such that when the guidewire 1212 is pulled back it is stopped at the vessel entry point by the retaining structure 1216. A dilator (not shown) may be advanced over the guidewire to dilate the opening. A double-barreled canula 1220 may be inserted over the guidewire. A first lumen 1222 of the canula 1220 may be advanced over the guidewire 1212. A second lumen 1224 of the canula 1220 may deliver the implant 904. As illustrated, a distal end of the second barrel 1224 of the canula 1220 may have a slight bend to facilitate placement of the implant 904. The implant 904 may be positioned at a distal end of the second barrel 1224 and discharged or ejected from the second barrel 1224 by a pusher 1224. The canula 1224 and the guidewire can be withdrawn from the opening, leaving the implant in place.

In FIG. 12A, the barbs 905 of the implant 904 are positioned in the implantation position. More specifically, when the implant 904 is within the lumen 1224 of the canula 1220, the barbs 905 are held in a folded position, bent toward the center of the implant 904, and substantially parallel to the housing 1102 of the implant 904. As the implant 904 is ejected from the lumen 1224 of the canula 1220, the barbs 905 spring from the implantation position to the securement position, as shown in FIG. 12B, extending away from the housing 1102 of the implant 904. FIG. 12B illustrates the implant 904 successfully implanted within the subcutaneous tissue 210 of a mammalian body, adjacent the blood vessel 110.

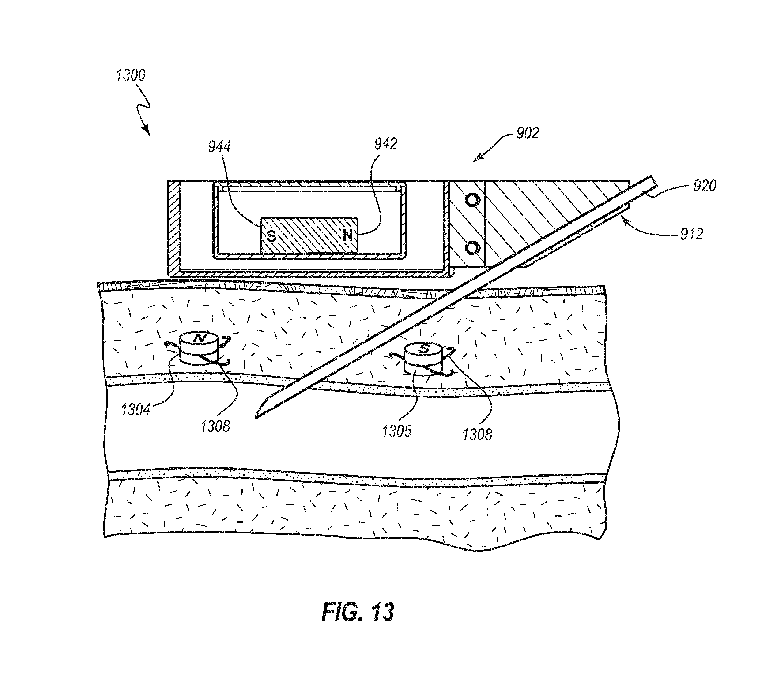

FIG. 13 is cross-section side view of another system 1300 for accessing a blood vessel 110 at a desired entry site. The system 1300 may include a detection and guide system 902 and a pair of implants 1304, 1305. The implants 1304, 1305 are positioned adjacent a blood vessel 110 at a distance from each other with the desired entry site between them. The detection and guide system 902 may include a locator 908 that is a bar magnet having a north pole 942 positioned at a first end and a south pole 944 positioned at a second end.

The implants 1304, 1305 also include magnetic material and may include barbs 1308 to secure the implants 1304, 1305 in their respective positions within subcutaneous tissue and/or adventitia within a mammalian body. In the illustrated embodiment, the implants 1302, 1304 may be configured similar to disk magnets having a north pole on a first side and a south pole on a second side. The first implant 1304 is oriented and positioned within the subcutaneous tissue 210 with the north pole facing upward, or toward the surface of the skin. The second implant 1305 is oriented and positioned within the subcutaneous tissue 210 with the south pole facing upward or toward the surface of the skin. Accordingly, the locator 908 aligns with the implants 1304, 1305 because the north pole 942 of the locator 908 attracts toward the south pole of the second implant 1305 and the south pole 944 of the locator 908 attracts toward the north pole of the first implant 1304. When the locator 908 is attracted by both implants 1304, 1305, it may remain in a generally fixed position between the implants 1304, 1305, regardless of minimal movements of the detection and guide system 902, thereby indicating both a position and a direction to align the needle guide 912.

In a preferred embodiment, the angle of the needle guide is adjustable based on a detected depth of the implant within the mammalian body.
In a preferred embodiment, the angle of the needle guide is fixed.
In a preferred embodiment, the implant is affixed within subcutaneous tissue of the mammalian body, adjacent the outer surface of the biological boundary structure.
In a preferred embodiment, the implant is affixed to adventitia of the biological boundary structure.
In a preferred embodiment, the detectable material of the implant comprises metal, and wherein the implant detector of the detection and guide system comprises a metal detector.
In a preferred embodiment, the metal detector comprises one or more metal detector coils within a coil housing, the one or more metal detector coils configured to detect the metal of the implant within the mammalian body.
In a preferred embodiment, wherein the metal detector is configured to produce an alternating current that passes through the one or more metal detector coils to produce an alternating magnetic field, such that when the coil housing is sufficiently close to the implant, the alternating magnetic field generated by the one or more metal detector coils produces eddy currents in the metal of the implant to produce a second alternating magnetic field that is detectable by the metal detector to detect the implant within the mammalian body.
In a preferred embodiment, the detection and guide system is calibrated to detect both a planar position and a depth of the implant.
In a preferred embodiment, the detectable material of the implant comprises a magnetic material that creates a detectable magnetic field.
In a preferred embodiment, the implant detector of the detection and guide system comprises a compass to detect the magnetic material of the implant, the compass including a locator configured to shift position relative to the housing and needle guide of the detection and guide system to align with a magnetic field of the magnetic material of the implant.
In a preferred embodiment, the locator comprises a magnet.
In a preferred embodiment, the locator is free floating within a housing of the compass.
In a preferred embodiment, the locator is in a liquid within a housing of the compass.
In a preferred embodiment, the locator rotates about an axis perpendicular to a bottom of the housing of the detection and guide system.
In a preferred embodiment, poles of the magnetic material of the implant are aligned parallel to a longitudinal axis of the biological boundary structure.
In a preferred embodiment, the magnetic material of the implant comprises a cylindrical magnet positioned within a housing of the implant.
In a preferred embodiment, the implant is a first implant and the system further comprises a second implant formed of a biocompatible material and configured to be implanted within the mammalian body proximate an outer surface of the biological boundary structure at a distance from the first implant in a direction along a longitudinal axis of the biological boundary structure, the second implant including a magnetic material, such that the locator aligns with the first implant and the second implant when positioned within a magnetic field between the first implant and second implant.
In a preferred embodiment, a north pole of the magnetic material of the first implant is configured facing outward toward the outside surface of the mammalian body and a south pole of the magnetic material of the second implant is facing outward toward the outside surface of the mammalian body, such that a south pole of the locator aligns with the first implant and a north pole of the locator aligns with the second implant.
Particular aspects of the invention are described in the following numbered statements.
1. A system for accessing a biological boundary structure at a desired entry site within a mammalian body, the system comprising:
   an implant formed of a biocompatible material and configured to be implanted within the mammalian body proximate an outer surface of the biological boundary structure, the implant including a detectable material that can be detected from outside the mammalian body; and
   a detection and guide system configured to be positioned outside the mammalian body to detect the implant and guide a needle to the desired entry site of the biological boundary structure, the detection system comprising:
      an implant detector positioned within a housing of the detection and guide system and configured to detect the detectable material of the implant within the mammalian body from outside the mammalian body; and
      a needle guide coupled to the housing of the detection unit and directed at an angle such that positioning the implant detector over the implant aligns an opening through the needle guide with the desired entry site.
2. The system of statement 1, wherein the angle of the needle guide is adjustable based on a detected depth of the implant within the mammalian body.
3. The system of statement 1, wherein the angle of the needle guide is fixed.
4. The system of statement 1, wherein the implant is affixed within subcutaneous tissue of the mammalian body, adjacent the outer surface of the biological boundary structure.
5. The system of statement 1, wherein the implant is affixed to adventitia of the biological boundary structure.
6. The system of statement 1, wherein the detectable material of the implant comprises metal, and wherein the implant detector of the detection and guide system comprises a metal detector.
7. The system of statement 6, wherein the metal detector comprises one or more metal detector coils within a coil housing, the one or more metal detector coils configured to detect the metal of the implant within the mammalian body.
8. The system of statement 7, wherein the metal detector is configured to produce an alternating current that passes through the one or more metal detector coils to produce an alternating magnetic field, such that when the coil housing is sufficiently close to the implant, the alternating magnetic field generated by the one or more metal detector coils produces eddy currents in the metal of the implant to produce a second alternating magnetic field that is detectable by the metal detector to detect the implant within the mammalian body.
9. The system of statement 8, wherein the detection and guide system is calibrated to detect both a planar position and a depth of the implant.
10. The system of statement 1, wherein the detectable material of the implant comprises a magnetic material that creates a detectable magnetic field.
11. The system of statement 10, wherein the implant detector of the detection and guide system comprises a compass to detect the magnetic material of the implant, the compass including a locator configured to shift position relative to the housing and needle guide of the detection and guide system to align with a magnetic field of the magnetic material of the implant.
12. The system of statement 11, wherein the locator comprises a magnet.
13. The system of statement 11, wherein the locator is free floating within a housing of the compass.
14. The system of statement 13, wherein the locator is in a liquid within a housing of the compass.
15. The system of statement 11, wherein the locator rotates about an axis perpendicular to a bottom of the housing of the detection and guide system.
16. The system of statement 11, wherein poles of the magnetic material of the implant are aligned parallel to a longitudinal axis of the biological boundary structure.
17. The system of statement 16, wherein the magnetic material of the implant comprises a cylindrical magnet positioned within a housing of the implant.
18. The system of statement 11, wherein the implant is a first implant and the system further comprises a second implant formed of a biocompatible material and configured to be implanted within the mammalian body proximate an outer surface of the biological boundary structure at a distance from the first implant in a direction along a longitudinal axis of the biological boundary structure, the second implant including a magnetic material, such that the locator aligns with the first implant and the second implant when positioned within a magnetic field between the first implant and second implant.
19. The system of statement 18, wherein a north pole of the magnetic material of the first implant is configured facing outward toward the outside surface of the mammalian body and a south pole of the magnetic material of the second implant is facing outward toward the outside surface of the mammalian body, such that a south pole of the locator aligns with the first implant and a north pole of the locator aligns with the second implant.
It will be understood by those having skill in the art that many changes may be made to the details of the above-described embodiments without departing from the underlying principles of the invention. The scope of the present invention should, therefore, be determined only by the following claims.

## Claims

1. A system for accessing a biological boundary structure at a desired entry site within a mammalian body, the system comprising:
an implant formed of a biocompatible material and configured to be implanted within the mammalian body proximate an outer surface of the biological boundary structure, the implant including a detectable material that can be detected from outside the mammalian body; and
a detection and guide system configured to be positioned outside the mammalian body to detect the implant and guide a needle to the desired entry site of the biological boundary structure, the detection system comprising:
an implant detector positioned within a housing of the detection and guide system and configured to detect the detectable material of the implant within the mammalian body from outside the mammalian body; and
a needle guide coupled to the housing of the detection unit and directed at an angle such that positioning the implant detector over the implant aligns an opening through the needle guide with the desired entry site.

2. The system of claim 1, wherein the angle of the needle guide is adjustable based on a detected depth of the implant within the mammalian body.

3. The system of claim 1, wherein the angle of the needle guide is fixed.

4. The system of claim 1, wherein the implant is affixed within subcutaneous tissue of the mammalian body, adjacent the outer surface of the biological boundary structure.

5. The system of claim 1, wherein the implant is affixed to adventitia of the biological boundary structure.

6. The system of claim 1, wherein the detectable material of the implant comprises metal,
wherein the implant detector of the detection and guide system comprises a metal detector, and
wherein the metal detector comprises one or more metal detector coils within a coil housing, the one or more metal detector coils configured to detect the metal of the implant within the mammalian body.

7. The system of claim 6, wherein the metal detector is configured to produce an alternating current that passes through the one or more metal detector coils to produce an alternating magnetic field, such that when the coil housing is sufficiently close to the implant, the alternating magnetic field generated by the one or more metal detector coils produces eddy currents in the metal of the implant to produce a second alternating magnetic field that is detectable by the metal detector to detect the implant within the mammalian body.

8. The system of claim 7, wherein the detection and guide system is calibrated to detect both a planar position and a depth of the implant.

9. The system of claim 1, wherein the detectable material of the implant comprises a magnetic material that creates a detectable magnetic field, and
wherein the implant detector of the detection and guide system comprises a compass to detect the magnetic material of the implant, the compass including a locator configured to shift position relative to the housing and needle guide of the detection and guide system to align with a magnetic field of the magnetic material of the implant.

10. The system of claim 9, wherein the locator comprises a magnet.

11. The system of claim 9, wherein the locator is free floating in a liquid within a housing of the compass.

12. The system of claim 9, wherein the locator rotates about an axis perpendicular to a bottom of the housing of the detection and guide system.

13. The system of claim 9, wherein poles of the magnetic material of the implant are aligned parallel to a longitudinal axis of the biological boundary structure and wherein the magnetic material of the implant comprises a cylindrical magnet positioned within a housing of the implant.

14. The system of claim 9, wherein the implant is a first implant and the system further comprises a second implant formed of a biocompatible material and configured to be implanted within the mammalian body proximate an outer surface of the biological boundary structure at a distance from the first implant in a direction along a longitudinal axis of the biological boundary structure, the second implant including a magnetic material, such that the locator aligns with the first implant and the second implant when positioned within a magnetic field between the first implant and second implant.

15. The system of claim 14, wherein a north pole of the magnetic material of the first implant is configured facing outward toward the outside surface of the mammalian body and a south pole of the magnetic material of the second implant is facing outward toward the outside surface of the mammalian body, such that a south pole of the locator aligns with the first implant and a north pole of the locator aligns with the second implant.
